# EUROPEAN PATENT SPECIFICATION

(11) **EP 4 702 926 B1**
(45) Date of publication and mention of the grant of the patent: **21.10.2026**
(21) Application number: 25196567.9
(22) Date of filing: 19.08.2025
(51) Int. Cl.: A61B 6/00, A61B 6/51

(54) **X-RAY IMAGING APPARATUS**
RÖNTGENBILDGEBUNGSVORRICHTUNG
APPAREIL D'IMAGERIE PAR RAYONS X

(30) Priority: 26.08.2024 KR 20240114043
(43) Date of publication of application: 04.03.2026
(73) Proprietor: Vatech Co., Ltd., Hwaseong-si, Gyeonggi-do 18449 (KR); Vatech Ewoo Holdings Co., Ltd., Hwaseong-si, Gyeonggi-do 18449 (KR)
(72) Inventor: Jeong, Jun Young, 18449 Hwaseong-si (KR)
(74) Representative: Lorenz & Kollegen

(56) References cited:
- US-A1- 2006 203 959
- US-A1- 2018 344 278

## Description

### CROSS REFERENCE TO RELATED APPLICATION

The present application claims priority to Korean Patent Application No. 10-2024-0114043, filed 26 August 2024.

### BACKGROUND

### Technical Field

The present disclosure relates to an X-ray imaging apparatus.

### Description of the Related Art

In order to accurately check an oral structure of a patient in a dental clinic and so on, an X-ray imaging apparatus is used. Such an X-ray imaging apparatus includes an X-ray generation apparatus configured to emit X-rays toward a subject, and includes an X-ray sensor (hereinafter, a sensor) configured to receive **X-**rays that pass through the subject. The X-ray imaging apparatus may be divided into an intraoral X-ray apparatus in which a sensor is disposed inside the oral cavity and X-ray imaging is performed on a portion inside the oral cavity by using X-rays generated from an X-ray generation apparatus positioned outside the oral cavity and an extraoral X-ray apparatus in which a sensor and an X-ray generation apparatus are disposed outside the oral cavity and X-ray imaging is performed on an overall structure of the oral cavity by rotating the sensor and the X-ray generation apparatus.

The X-ray apparatus positioned outside the oral cavity is also referred to as an extraoral X-ray apparatus, and a camera may be used together with X-rays for acquiring an optical image of an imaging subject. However, when the camera is positioned in a direction of a sensor part, there are limitations such as the camera being deteriorated by X-rays, the sensor part being excessively enlarged, and so on.

A technology which becomes a background of the present disclosure is disclosed in Korean Patent No. 10-0933198. A further example of a known X-ray imaging apparatus is shown in US2018/344278A1, which shows an X-ray imaging apparatus with an additional camera for an optical image.

### SUMMARY

Accordingly, the present disclosure has been made keeping in mind the above problems occurring in the related art, and an objective of the present disclosure is to provide an X-ray imaging apparatus.

However, the problems to be solved by the present disclosure are not limited to the above-described problems, and there may be other problems to be solved by the present disclosure.

As a technical mechanism for realizing the technical objective of the present disclosure, according to a first aspect of the present disclosure, there is provided an X-ray imaging apparatus including: an imaging part including a generator part and a sensor part that are facing each other with an imaging subject positioned therebetween; and a driving control part configured to rotate the generator part and the sensor part around a rotation axis positioned between the generator part and the sensor part, thereby radiographing an X-ray image of the imaging subject. Furthermore, the generator part includes an X-ray generation part configured to generate X-rays emitted toward the imaging subject by being controlled by the driving control part; and a camera part configured to photograph a three-dimensional (3D) optical image of the imaging subject by being controlled by the driving control part when the generator part and the sensor part are rotated.

According to an embodiment of the present disclosure, the camera part is disposed inside the guide groove that is recessed from an outer surface of the generator part to an inner portion of the generator part, and the X-ray generation part may be disposed on a first side of the guide groove.

The above-described technical solutions are set forth to illustrate only and should not be construed as intended to limit the present disclosure. In addition to the exemplary embodiments described above, additional embodiments may exist in the drawings and detailed description of the present disclosure.

According to the technical solution of the present disclosure described above, in the X-ray imaging apparatus according to the present disclosure, since a 3D camera is disposed on a first side of the X-ray generation part, a phenomenon in which the camera is deteriorated by X-rays may be prevented.

In addition, since the 3D camera is disposed in a tilted manner within a recessed portion (the guide groove) of the generator part, a focal distance and an optical axis between the 3D camera and the imaging subject are capable of being adjusted.

In addition, as a sensor for large FOV (Field of View) has become larger in area, a sensor part has insufficient remaining space. Furthermore, when a camera is mounted under such circumstances, a problem of the sensor part becoming excessively large size exists. However, in the present disclosure, since the 3D camera is disposed on the X-ray generation part and the focal distance and the optical axis are adjusted, more accurate 3D optical images are capable of being acquired.

However, the effects capable of being acquired from the present disclosure are not limited to the effects described above, and other effects may also exist.

### BRIEF DESCRIPTION OF THE DRAWINGS

The above and other objectives, features, and other advantages of the present disclosure will be more clearly understood from the following detailed description when taken in conjunction with the accompanying drawings, in which:
FIG. 1 is a view illustrating a conventional X-ray imaging apparatus;
FIG. 2 is a schematic view illustrating a generator part according to an embodiment of the present disclosure;
FIG. 3 is a cross-sectional view illustrating the generator part according to an embodiment of the present disclosure;
FIG. 4 is a schematic view illustrating a light source part according to an embodiment of the present disclosure; and
FIG. 5 is a schematic view illustrating a relationship between a camera, a sensor, and a rotation axis of an X-ray imaging apparatus according to an embodiment of the present disclosure.

### DETAILED DESCRIPTION

Hereinafter, embodiments of the present disclosure will be described in detail such that those skilled in the art to which the present disclosure belongs may easily implement the present disclosure with reference to the accompanying drawings.

However, the present disclosure may be implemented in many different forms and is not limited to the embodiments to be described herein. In addition, in order to clearly describe the present disclosure with reference to the drawings, parts irrelevant to the description are omitted, and similar reference numerals denote similar parts throughout the specification.

Throughout the present specification, when a portion is described to be "connected" to another portion, this includes not only a case where the portion is "directly connected" thereto, but also a case where the portion is "electrically connected" thereto with another element therebetween.

Throughout the present specification, when a member is referred to as being "on", "on an upper portion of", "on an upper end of", "under", "on a lower portion of", or "on a lower end of" another member, this may include not only the case where a member is in contact with another member, but also the case where another member exists between two members.

Throughout the present specification, when a part is referred to as "including" an element, it means that the part may include other elements as well without excluding the other elements unless specifically stated otherwise.

Hereinafter, an X-ray imaging apparatus according to an embodiment of the present disclosure will be described.

As a technical mechanism for realizing the technical objective of the present disclosure, according to a first aspect of the present disclosure, there is provided an X-ray imaging apparatus including: an imaging part including a generator part and a sensor part that are facing each other with an imaging subject positioned therebetween; and a driving control part configured to rotate the generator part and the sensor part around a rotation axis positioned between the generator part and the sensor part, the driving control part being configured to radiograph an X-ray image of the imaging subject by controlling the generator part and the sensor part. Furthermore, the generator part includes an X-ray generation part configured to generate X-rays emitted toward the imaging subject, and includes a camera part disposed on a first side of the X-ray generation part. Furthermore, the driving control part is configured to photograph a three-dimensional optical image of the imaging subject by controlling the camera part while the generator part and the sensor part are rotated.

That is, the X-ray imaging apparatus relates to an extraoral X-ray apparatus including: the imaging part; the sensor part disposed such that the sensor part is spaced apart from the imaging part with a predetermined distance so that the imaging subject is capable of being disposed between the imaging part and the sensor part; and a control part (the driving control part) configured to control an operation of the imaging part and the sensor part. Furthermore, the imaging part includes the X-ray generation part configured to generate X-rays, the camera part disposed on the first side of the X-ray generation part, and the generator part on which the X-ray generation part and the camera part are disposed. Furthermore, the X-ray generation part and the camera part are disposed on a surface of the generator part.

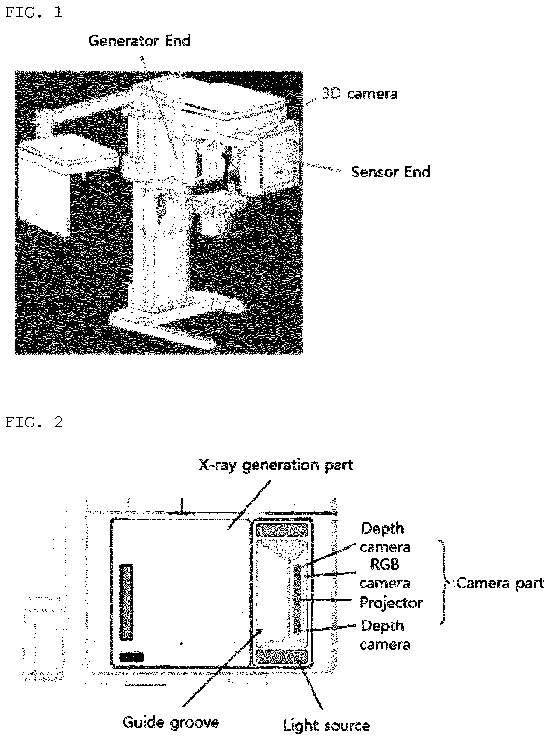

FIG. 1 is a view illustrating an X-ray imaging apparatus according to an embodiment of the present disclosure. The X-ray imaging apparatus according to an embodiment of the present disclosure includes the imaging part, the driving control part configured to drive and control the imaging part, and an image processing part configured to reconstruct an X-ray image from an imaging result of the imaging part.

Referring to FIG. 1, the imaging part includes a generator end (the generator part of the present disclosure) and a sensor end (the sensor part of the present disclosure) that are facing each other with the imaging subject positioned therebetween. The generator end is provided with the X-ray generation part configured to generate X-rays and to emit the X-rays toward the imaging subject, and is provided with the camera part including a 3D camera. Furthermore, the sensor part is provided with an X-ray sensor configured to detect X-rays that pass through the imaging subject.

At this time, by tilting the camera part within a guide groove of the generator part, a focal distance and an optical axis condition may be satisfied.

The generator part may include a frame connecting the X-ray generation part and the camera part to each other.

The X-ray generation part is a portion that generates X-rays, and the X-rays are generated by a thermionic emission method or a field emission method. The X-rays generated in the X-ray generation part pass through the imaging subject and are detected in the sensor part.

The driving control part drives and controls the imaging part in order to radiograph an X-ray image and/or a 3D optical image for the imaging subject. To this end, the driving control part rotates the generator part and the sensor part with respect to the rotation axis between the generator part and the sensor part, and controls the X-ray generation part, the 3D camera, and the X-ray sensor so as to acquire an X-ray image and/or a 3D optical image for the imaging subject.

The driving control part may be in the form of a firmware controlling the generator part, the sensor part, and the driving part.

An X-ray detection result detected by the X-ray sensor and an optical image acquired by the 3D camera are reconstructed into an X-ray image and a 3D optical image of the imaging subject through the image processing part. At this time, the image processing part may have a form of an algorithm in which an image is reconstructed on the basis of the X-ray detection result obtained by sensing from the sensor part.

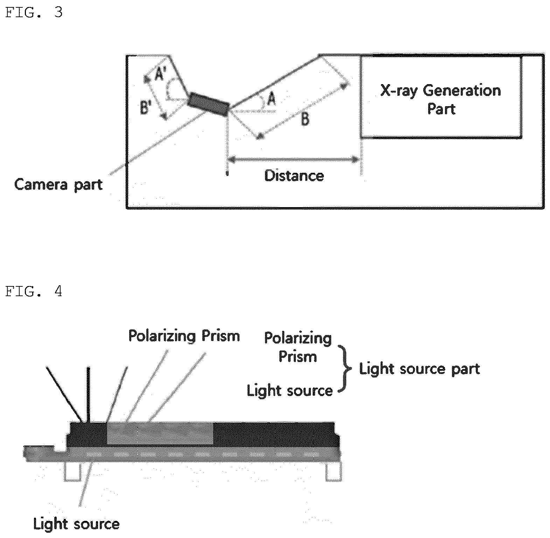

FIG. 2 is a schematic view illustrating the generator part according to an embodiment of the present disclosure, and FIG. 3 is a cross-sectional view illustrating the generator part according to an embodiment of the present disclosure.

According to an embodiment of the present disclosure, the generator part may include the guide groove recessed from an outer surface to an inner surface, but is not limited thereto. As will be described later, the guide groove is provided for tilting and positioning the camera part, and has an irregular quadrilateral shape with different lengths of opposing sides and different angle sizes.

According to an embodiment of the present disclosure, the camera part is disposed inside the guide groove, and the X-ray generation part may be disposed on a first side of the guide groove.

According to an embodiment of the present disclosure, the guide groove may be formed such that an inclination angle of a direction (a first direction) in which the X-ray generation part is positioned is smaller than an inclination angle of a direction (a second direction) in which the X-ray generation part is not positioned. That is, the camera part mounted on a distal end of the guide groove may be configured such that the camera part faces the rotation axis described later, but is not limited thereto.

According to an embodiment of the present disclosure, the guide groove may be formed in consideration of a focal distance and an optical axis between the camera part and the imaging subject, but is not limited thereto.

According to an embodiment of the present disclosure, the guide groove may be formed such that the camera part faces the rotation axis in consideration of a focal distance and an optical axis between the camera part and the imaging subject, but is not limited thereto.

Referring to FIG. 2, it may be confirmed that the X-ray generation part is disposed inside the generator part and the camera part is disposed inside the guide groove after the guide groove is formed in the first side (3 o'clock direction in FIG. 2) of the X-ray generation part.

At this time, the guide groove of the generator part is formed according to the cross-sectional view in FIG. 3. For example, referring to FIG. 3, an inclination angle A within the guide groove in a direction in which the X-ray generation part is positioned is smaller than an inclination angle A' (based on an acute angle) in a direction opposite to the X-ray generation part. In addition, among sides constituting the guide groove, a length B of a side facing the X-ray generation part may be formed longer than a length B' of a side disposed in a direction opposite to the X-ray generation part. In this case, A may be an inverse proportional relationship and B may be a proportional relationship with respect to the distance between the generator part and the camera part. However, since the guide groove is formed in consideration of the focal distance and the optical axis between the camera part and the imaging subject, the shape of the guide groove may be adjusted as required.

According to an embodiment of the present disclosure, the camera part may be disposed such that the camera part is tilted at a predetermined angle within the guide groove, but is not limited thereto.

The predetermined angle may be 10° to 80°, but is not limited thereto. The predetermined angle may be determined by a size of the camera part, a depth of the guide groove, a distance between the camera part and the X-ray generation part, a type of light source, a distance between the imaging subject and the camera part, and so on.

When the camera part is not arranged in a tilted manner within the guide groove, the camera part not only fails to secure sufficient focal distance but also captures a side surface of the imaging subject obliquely rather than a front surface of the imaging subject. In order to overcome this problem, the camera part is mounted inside the generator part so that a sufficient focal distance is secured, and the camera part is tilted at the predetermined angle so that the camera part is directed toward the rotation axis by using the shape of the guide groove, so that the camera part is capable of photographing the front surface of the imaging subject with the accurate focal distance.

According to an embodiment of the present disclosure, the X-ray imaging apparatus may additionally include a light source part disposed in a first side of the camera part, a second side of the camera part, or a direction orthogonal to the first direction in which the X-ray generation part is positioned, but is not limited thereto.

As can be seen in FIG. 2, the light source part of the X-ray imaging apparatus may be formed on an outer circumference of the guide groove in which the camera part is mounted. As illustrated in FIG. 2, when a guide groove is mounted on a right side (3 o'clock direction) of the X-ray generation part, the light source part may be disposed on an upper side and a lower side (12 o'clock direction and 6 o'clock direction) of the guide groove, but may be disposed between the guide groove and the X-ray generation part.

According to an embodiment of the present disclosure, the light source part may include a light source configured to emit light to the imaging subject, and may include a polarizing prism for matching an optical axis of the light source with an optical axis of the camera part, but is not limited thereto.

The light source is configured to emit light to the imaging subject so as to assist the camera part to receive sufficient light while the camera part performs photographing, and the polarizing prism is configured to match the optical axis of the light source with the optical axis of the camera part.

According to an embodiment of the present disclosure, the camera part may include the 3D camera. Furthermore, the 3D camera may include an RGB camera configured to photograph a surface of the imaging subject, and may include at least one depth camera configured to photograph a depth of the imaging subject, but is not limited thereto.

According to an embodiment of the present disclosure, the camera part may include an RGB camera configured to acquire color information of the imaging subject, and may include a depth camera configured to acquire depth information of a surface of the imaging subject. Furthermore, a color 3D facial optical image for the imaging subject may be acquired by using the color information and the depth information acquired by the RGB camera and the depth camera, but is not limited thereto. In addition, in order to acquire more accurate depth information, the camera part may include a plurality of depth cameras spaced apart from each other, and may additionally include a projector capable of providing a predetermined pattern to the surface of the imaging subject.

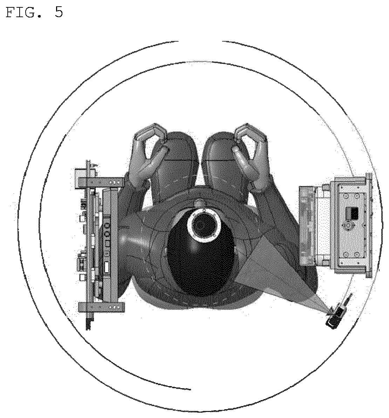

FIG. 5 is a schematic view illustrating a relationship between a camera, a sensor, and a rotation axis of an X-ray imaging apparatus according to an embodiment of the present disclosure.

In the X-ray imaging apparatus, specifically the extraoral X-ray imaging apparatus, the X-ray generation part of the generator part and the X-ray sensor of the sensor part are facing each other with the rotation axis positioned therebetween, and the rotation axis passes through the imaging subject.

In the X-ray imaging apparatus according to the present disclosure, the camera part is tilted such that the camera positioned in the generator part faces the rotation axis, so that the camera may view the imaging subject in a straight (vertical) direction. Therefore, in the X-ray imaging apparatus, the generator, the sensor, and the camera may all be disposed so that the generator, the sensor, and the camera face the rotation axis.

Here, the camera may be disposed in the generator part and the focal distance may be adjusted, and the guide groove in which the camera is disposed is provided for correcting the view angle of the camera.

The aforementioned description of the present disclosure is used for exemplification, and it can be understood by those skilled in the art that the present disclosure can be easily modified in other detailed forms without changing the technical spirit or requisite features of the present disclosure. Therefore, it should be appreciated that the aforementioned exemplary embodiments are illustrative in all aspects and are not restricted. For example, respective constituent elements described as single types can be distributed and implemented and, similarly, constituent elements described to be distributed can also be implemented in a coupled form.

## Claims

1. An X-ray imaging apparatus comprising:
an imaging part comprising a generator part and a sensor part facing each other with an imaging subject positioned therebetween; and
a driving control part configured to rotate the generator part and the sensor part around a rotation axis positioned between the generator part and the sensor part, thereby radiographing an X-ray image of the imaging subject,
wherein the generator part comprises:
an X-ray generation part configured to generate X-rays emitted toward the imaging subject by being controlled by the driving control part; and
a camera part configured to photograph a three-dimensional (3D) optical image of the imaging subject by being controlled by the driving control part when the generator part and the sensor part are rotated,
**characterized in that** the camera part is disposed within a guide groove recessed from an outer surface of the generator part to an inner portion of the generator part, and the X-ray generation part is disposed on a first side of the guide groove.

2. The X-ray imaging apparatus of claim 1, wherein the guide groove is formed such that an inclination angle of a direction (a first direction) in which the X-ray generation part is positioned is smaller than an inclination angle of a direction (a second direction) in which the X-ray generation part is not positioned.

3. The X-ray imaging apparatus of claim 1, wherein the guide groove is formed such that the camera part faces the rotation axis in consideration of a focal distance and an optical axis between the camera part and the imaging subject.

4. The X-ray imaging apparatus of claim 1, wherein the camera part is disposed in a tilted at a predetermined angle within the guide groove.

5. The X-ray imaging apparatus of claim 1, further comprising:
a light source part disposed in a first side of the camera part, a second side of the camera part, or a direction orthogonal to a first direction in which the X-ray generation part is positioned.

6. The X-ray imaging apparatus of claim 5, wherein the light source part comprises a light source configured to emit light to the imaging subject, and comprises a polarizing prism for matching an optical axis of the light source with an optical axis of the camera part.

7. The X-ray imaging apparatus of claim 1, wherein the camera part comprises a 3D camera, and the 3D camera comprises an RGB camera configured to photograph a surface of the imaging subject and at least one depth camera configured to photograph a depth of the imaging subject.

## Patentansprüche

1. Röntgenbildgebungsvorrichtung, umfassend:
einen Bildgebungsteil, der einen Generatorteil und einen Sensorteil umfasst, die einander gegenüberliegen, wobei sich ein Bildgebungsobjekt zwischen ihnen befindet; und
einen Ansteuerungs- und Steuerungsteil, der so ausgelegt ist, dass er den Generatorteil und den Sensorteil um eine zwischen dem Generatorteil und dem Sensorteil angeordnete Drehachse dreht, wodurch ein Röntgenbild des Bildgebungsobjekts aufgenommen wird,
wobei der Generatorteil umfasst:
einen Röntgenstrahl-Erzeugungsteil, der so ausgelegt ist, dass er unter Steuerung durch den Antriebssteuerungsteil Röntgenstrahlen erzeugt, die in Richtung des Abbildungsobjekts emittiert werden; sowie
einen Kamerateil, der so ausgelegt ist, dass er unter Steuerung durch den Antriebssteuerungsteil ein dreidimensionales (3D) optisches Bild des Abbildungsobjekts aufnimmt, wenn der Generatorteil und der Sensorteil gedreht werden,
**dadurch gekennzeichnet, dass** der Kamerateil in einer Führungsnut angeordnet ist, die von einer Außenfläche des Generatorteils bis in einen inneren Bereich des Generatorteils vertieft ist, und dass der Röntgenstrahlerzeugungsteil auf einer ersten Seite der Führungsnut angeordnet ist.

2. Röntgenbildgebungsvorrichtung nach Anspruch 1, wobei die Führungsnut so ausgebildet ist, dass ein Neigungswinkel einer Richtung (einer ersten Richtung), in der der Röntgengenerator angeordnet ist, kleiner ist als ein Neigungswinkel einer Richtung (einer zweiten Richtung), in der der Röntgengenerator nicht angeordnet ist.

3. Röntgenbildgebungsvorrichtung nach Anspruch 1, wobei die Führungsnut so ausgebildet ist, dass das Kamerateil unter Berücksichtigung einer Brennweite und einer optischen Achse zwischen dem Kamerateil und dem abzubildenden Objekt der Drehachse zugewandt ist.

4. Röntgenbildgebungsvorrichtung nach Anspruch 1, wobei das Kamerateil innerhalb der Führungsnut um einen vorbestimmten Winkel geneigt angeordnet ist.

5. Röntgenbildgebungsvorrichtung nach Anspruch 1, ferner umfassend:
einen Lichtquellenteil, der an einer ersten Seite des Kamerateils, an einer zweiten Seite des Kamerateils oder in einer Richtung orthogonal zu einer ersten Richtung, in der der Röntgenstrahlerzeugungsteil angeordnet ist, angeordnet ist.

6. Röntgenbildgebungsvorrichtung nach Anspruch 5, wobei der Lichtquellenteil eine Lichtquelle umfasst, die so konfiguriert ist, dass sie Licht auf das abzubildende Objekt ausstrahlt, und ein Polarisationsprisma umfasst, um eine optische Achse der Lichtquelle mit einer optischen Achse des Kamerateils in Übereinstimmung zu bringen.

7. Röntgenbildgebungsvorrichtung nach Anspruch 1, wobei der Kamerateil eine 3D-Kamera umfasst und die 3D-Kamera eine RGB-Kamera, die so konfiguriert ist, dass sie eine Oberfläche des Bildgebungsobjekts aufnimmt, sowie mindestens eine Tiefenkamera umfasst, die so konfiguriert ist, dass sie eine Tiefe des Bildgebungsobjekts aufnimmt.

## Revendications

1. Appareil d'imagerie à rayons X comprenant :
une partie d'imagerie comprenant une partie génératrice et une partie capteur se faisant face, un sujet d'imagerie étant positionné entre celles-ci ; et
une partie de commande de pilotage configurée pour faire tourner la partie génératrice et la partie capteur autour d'un axe de rotation situé entre la partie génératrice et la partie capteur, afin de réaliser ainsi une radiographie du sujet d'imagerie,
dans lequel la partie génératrice comprend :
une partie de génération de rayons X configurée pour générer des rayons X émis vers le sujet à imager en étant commandée par la partie de commande d'entraînement ; et
une partie caméra configurée pour photographier une image optique tridimensionnelle (3D) du sujet à imager sous le contrôle de la partie de commande d'entraînement lorsque la partie génératrice et la partie capteur sont mises en rotation,
**caractérisé en ce que** la partie caméra est disposée à l'intérieur d'une rainure de guidage en retrait par rapport à une surface extérieure de la partie génératrice vers une partie intérieure de celle-ci, et la partie de génération de rayons X est disposée sur un premier côté de la rainure de guidage.

2. Appareil d'imagerie à rayons X selon la revendication 1, dans lequel la rainure de guidage est formée de telle sorte que l'angle d'inclinaison d'une direction (une première direction) dans laquelle la partie de génération de rayons X est positionnée soit inférieur à l'angle d'inclinaison d'une direction (une deuxième direction) dans laquelle la partie de génération de rayons X n'est pas positionnée.

3. Appareil d'imagerie à rayons X selon la revendication 1, dans lequel la rainure de guidage est formée de telle sorte que la partie caméra soit orientée vers l'axe de rotation, en tenant compte de la distance focale et de l'axe optique entre la partie caméra et le sujet à imager.

4. Appareil d'imagerie à rayons X selon la revendication 1, dans lequel la partie caméra est disposée de manière inclinée selon un angle prédéterminé à l'intérieur de la rainure de guidage.

5. Appareil d'imagerie à rayons X selon la revendication 1, comprenant en outre :
une partie source de lumière disposée sur un premier côté de la partie caméra, sur un deuxième côté de la partie caméra, ou dans une direction orthogonale à une première direction dans laquelle est positionnée la partie génératrice de rayons X.

6. Appareil d'imagerie à rayons X selon la revendication 5, dans lequel la partie source de lumière comprend une source de lumière configurée pour émettre de la lumière vers le sujet à imager, et comprend un prisme polarisant destiné à aligner l'axe optique de la source de lumière avec l'axe optique de la partie caméra.

7. Appareil d'imagerie à rayons X selon la revendication 1, dans lequel la partie caméra comprend une caméra 3D, et la caméra 3D comprend une caméra RVB configurée pour photographier une surface du sujet à imager et au moins une caméra de profondeur configurée pour photographier une profondeur du sujet à imager.
